(19) Europäisches Patentamt / European Patent Office / Office européen des brevets

(11) **EP 2 363 418 B1**

(12) **EUROPEAN PATENT SPECIFICATION**

(45) Date of publication and mention
of the grant of the patent:
**08.02.2017 Bulletin 2017/06**

(51) Int Cl.:
*C07K 16/28* (2006.01)    *C12N 5/09* (2010.01)
*C12N 5/18* (2006.01)    *C12P 21/08* (2006.01)

(21) Application number: **11155076.0**

(22) Date of filing: **22.10.2003**

(54) **Method of obtaining recombinant mammalian myeloma cell lines adapted to growth in serum- and protein-free media**

Verfahren zum Erhalt von rekombinanten Säugetier-Myolemzelllinien, die für ein Wachstum in serum- und proteinfreien Medien angepasst sind

Procédé pour l'obtention de lignées cellulaires de myélome de mammifère recombinantes adaptées pour se développer dans des milieux sans protéines et sans sérum

(84) Designated Contracting States:
**AT BE BG CH CY CZ DE DK EE ES FI FR GB GR HU IE IT LI LU MC NL PT RO SE SI SK TR**

(30) Priority: **23.10.2002 CU 23920020**

(43) Date of publication of application:
**07.09.2011 Bulletin 2011/36**

(62) Document number(s) of the earlier application(s) in accordance with Art. 76 EPC:
**03757654.3 / 1 564 285**

(73) Proprietor: **Centro de Inmunologia Molecular
12100 Ciudad Habana (CU)**

(72) Inventors:
• **Perez Rodriguez, Rolando**
  **10500 Ciudad Habana (CU)**
• **Castillo Vitlloch, Adolfo**
  **13600 Ciudad Habana (CU)**
• **Vitores Sarazola, Svieta**
  **12200 Ciudad Habana (CU)**
• **Boggiano Ayo, Tammy**
  **12100 Ciudad Habana (CU)**
• **Rojas Del Calvo, Luis**
  **12100 Ciudad Habana (CU)**

(74) Representative: **Schiweck, Weinzierl & Koch
European Patent Attorneys
Landsberger Straße 98
80339 München (DE)**

(56) References cited:
**EP-A1- 0 712 863    WO-A2-96/08565**

• **CASTILLO A J ET AL: "Adaptation and selection of NS0 myeloma cell lines producing recombinant monoclonal antibodies in protein-free medium", ANIMAL CELL TECHNOLOGY: FROM TARGET TO MARKET: PROCEEDINGS OF THE 17TH ESACT MEETING, TYLÖSAND, SWEDEN, JUNE 10-14, 2001, KLUWER ACADEMIC PUBLISHERS, 10 June 2001 (2001-06-10), pages 160-163, XP009121996, ISBN: 978-1-4020-0264-9**
• **VAN ERP R ET AL: "Monitoring of the production of monoclonal antibodies by hybridomas. Part I: Long-term cultivation in hollow fibre bioreactors using serum-free medium", JOURNAL OF BIOTECHNOLOGY, ELSEVIER SCIENCE PUBLISHERS, AMSTERDAM, NL, vol. 20, no. 3, 1 October 1991 (1991-10-01), pages 235-248, XP023944485, ISSN: 0168-1656, DOI: 10.1016/0168-1656(91)90285-4 [retrieved on 1991-10-01]**
• **KEEN M J ET AL: "ADAPTATION OF CHOLESTEROL-REQUIRING NS0 MOUSE MYELOMA CELLS TO HIGH DENSITY GROWTH IN A FULLY DEFINED PROTEIN-FREE AND CHOLESTEROL-FREE CULTURE MEDIUM", CYTOTECHNOLOGY, KLUWER ACADEMIC PUBLISHERS, DORDRECHT, NL, vol. 17, 1 January 1995 (1995-01-01), pages 203-211, XP009037177, ISSN: 0920-9069, DOI: 10.1007/BF00749658**

- SINACORE M S ET AL: "ADAPTATION OF MAMMALIAN CELLS TO GROWTH IN SERUM-FREE MEDIA", MOLECULAR BIOTECHNOLOGY, HUMANA PRESS, INC, US, vol. 15, no. 3, 1 July 2000 (2000-07-01), pages 249-257, XP009001160, ISSN: 1073-6085, DOI: 10.1385/MB:15:3:249
- GORFIEN S ET AL: "Growth of NS0 cells in protein-free, chemically defined medium", BIOTECHNOLOGY PROGRESS, AMERICAN INSTITUTE OF CHEMICAL ENGINEERS, US, vol. 16, no. 5, 1 September 2000 (2000-09-01), pages 682-687, XP002350529, ISSN: 8756-7938, DOI: 10.1021/BP000109A
- MATEO C ET AL: "Humanization of a mouse monoclonal antibody that blocks the epidermal growth factor receptor: recovery of antagonistic activity", IMMUNOTECHNOLOGY, ELSEVIER SCIENCE PUBLISHERS BV, NL, vol. 3, no. 1, 1 March 1997 (1997-03-01), pages 71-81, XP004075440, ISSN: 1380-2933, DOI: 10.1016/S1380-2933(97)00065-1
- CROMBET-RAMOS T ET AL: "Antiproliferative angiogenetic and proapoptotic activity of H-R3: A humanized anti-EGFR antibody", INTERNATIONAL JOURNAL OF CANCER, JOHN WILEY & SONS, INC, UNITED STATES, SWITZERLAND, GERMANY, vol. 101, no. 6, 20 October 2002 (2002-10-20), pages 567-575, XP008104826, ISSN: 0020-7136, DOI: 10.1002/IJC.10647 [retrieved on 2002-08-27]
- BAKER K N ET AL: "Metabolic control of recombinant protein N-glycan processing in NS0 and CHO cells", BIOTECHNOLOGY AND BIOENGINEERING, WILEY & SONS, HOBOKEN, NJ, US, vol. 73, no. 3, 5 May 2001 (2001-05-05), pages 188-202, XP002524941, ISSN: 0006-3592, DOI: 10.1002/BIT.1051

**Description**

**Field of the Invention:**

The present invention relates to biotechnology, specifically to a method of recovering stable cell clones adapted to serum- and protein-free medium by a two-stage adaptation process.

**Background of the invention:**

Since the development of the in vitro cultivation of mammalian cells the demand for large scale production of these cells has increased due to diagnostic and therapeutic potential of many of the products they produce. These useful agents include monoclonal antibodies, human growth hormone, lymphokines, erythropoietin, blood clotting factors and tissue plasminogen activators.

The use of recombinant monoclonal antibodies (rMab) for therapy and *in vivo* diagnosis of different diseases imply in many cases the use of high dose treatments. This fact makes necessary the production of large amount of the rMab of interest with a very high purity.

Several recombinant monoclonal antibodies with potential use in cancer and autoimmune diseases therapy and diagnostic have been expressed in NSO myeloma cells at Center of Molecular Immunology. US 5,891,996, describe the obtainment of the chimeric and humanized antibodies against Epidermal Growth Factor receptor (EGF-R), useful in diagnosis and therapy of tumors expressing said receptor. WO 97/19111 describes anti-CD6 monoclonal antibodies useful in diagnosis and therapy in patients suffering psoriasis. Gavilondo et al. in Hybridoma 9 No.5, 1999 reported an anti-CD3 monoclonal antibody called IOR-T3a

For protein-free culturing conditions, various techniques have been developed. Thus, specifically defined, complete protein-free media have been developed which allow the cell growth under protein-free conditions. WO 97/05240 describes the expression of recombinant proteins under protein-free conditions.

JP 2696001 describes the use of a protein-free medium for the production of factor VIII in CHO cells by adding a non-ionic surface-active agent or cyclodextrin to increase the productivity of the host cells. To increase the effectiveness of these additives, the addition of, e.g., butyrate and lithium is recommended.

WO 96/26266 describes the culturing of cells in a medium which contains a glutamin-containing protein hydrolysate whose content of free amino acids is less than 15% of the total weight of the protein, and whose peptides have a molecular weight of less than 44 kD. As the culturing medium for the cell cultures, a synthetic minimum medium is used as the basic medium to which, inter alia, fetal calf serum, gentamycine and mercapto-ethanol are added in addition to protein hydrolysate. The use of this serum-containing medium for the recombinant production of blood factors has not been mentioned.

U.S. Pat. No. 5,393,668 A describes special synthetic surfaces which allow the growth of adherent cells under protein-free conditions.

To stimulate cell proliferation, CHO cells which over express human insulin have been multiplied on an artificial substrate to which insulin is covalently bound (Ito et al. 1996 PNAS U.S.A. 93:3598-3601).

Reiter et al. (1992. Cytotechnology 9:247-253) describe the immobilisation of r-CHO cells first grown in serum-containing medium at a high density on carriers, and subsequent perfusion of the immobilized cells in protein-free medium during the production phase, wherein a continuous liberation of protein into the cell culture supernatant was found. There, the cells were maintained for less than 10 generations in protein-free medium.

Previous methods for the successful preparation of a large-scale cell culture under protein-free conditions have been described for continuous cell lines; in particular VERO cells (WO 96/15231). There, the cells are grown under serum- and protein-free conditions from the original ampoule up to a large technical scale of 1200 liters.

To adapt cells initially grown under serum-containing conditions to protein free medium is a rather troublesome process which usually takes long time; in addition, it has repeatedly been found that the yield of expressed protein and the productivity of recombinant CHO cells greatly drops after adaptation in protein-free medium as compared to serum-containing conditions (Paterson et al. 1994. Appl. Microbiol. Biotechnol. 40:691-658). This is the consequence of an instability or reduced growth of the recombinant clones due to the changed culturing conditions. Despite the use of a stable original clone, on account of the altered fermentation conditions, repeatedly a large portion of the cells become cells with reduced expression or also non-producers, which overgrow product producers during the production process, whereby the culture of the fermenter finally largely consists of non-producers or of such cells having a low expression. In the present invention we have established an approach to develop stable cell lines adapted to serum- and protein-free media. Following this approach several clones were isolated in protein-free medium.

**Detailed Description of the Invention:**

*Two stage adaptation of cell lines to protein free medium.*

This procedure comprises mammalian cell lines, for which it's not possible to carry out a direct procedure of adaptation from serum-supplemented or serum-free medium to protein-free medium.

The method of the present invention consists of a two stages process during adaptation of cell lines to protein-free medium (PFM).

The first step which is consider as a Non critical stage: the reduction of protein contents occurs without the lost of cell viability and there is not an important decrease of population doubling time in each step of protein concentration. The non-critical stage is observed usually between 5 and 0.5 mg/mL of total protein concentration in the culture medium and the culture shows almost the same growth rate that in the initial culture medium.

This first stage starts with cell line viability between 80 and 100% and cells are grown in culture media with consecutive protein concentration reduction up to a critical protein concentration at which cell viability drop to 0%. This protein concentration is the start point for the next stage.

The second step which is consider as a Critical stage: At this stage it occurs a decrease in cell viability and population-doubling time of the cells and it will take more time to adapt from one step of protein concentration to another. There exist critical protein concentrations in the culture medium, which is not possible to bypass during the adaptation process. These critical protein concentrations are specific for each recombinant cell line, but usually are below 0.6 mg/mL. Once the cells have recovered the initial viability and growth rate at these critical protein concentrations there is possible to subculture to the follow condition with lower protein concentration.

Once the critical protein concentration is fixed, its closest higher protein concentration which support cells growth is consider the pre-critical protein concentration. Starting from the pre-critical protein concentration it is reduced slowly up to cells recover the initial viability and growth rate.

The selected combination of steps to reduce the protein concentration at the critical stage will determine the total adaptation time in this stage and the rate of adaptation ($V_{adapt.}$), calculated as the relationship:

$$V_{adapt.} = \frac{\Delta \text{ Protein concentration}}{\Delta T_{adapt.}}$$

However this step combination will not have influence upon the time needed for adaptation to each protein concentration, including critical concentrations.

In order to determine the end of non-critical stage and the critical protein concentrations it's necessary to carry out an stepwise adaptation, by serial dilutions of the cell culture in the desired protein free medium reducing two fold the protein concentration each time (Table 1). This reduction can be done by the decrease of serum concentration or supplementing the basal medium with different levels of some rich in proteins serum substitute.

*Table 1: Stepwise reduction of protein concentration in order to determine critical concentrations starting from a culture medium supplemented with 5 mg/mL of protein (equivalent to 10 % of fetal bovine serum).*

| Step Number | Total protein concentration mg/mL | Equivalent FBS concentration*, % v/v |
|---|---|---|
| 1 | 5.000 | 10.00 |
| 2 | 2.500 | 5.00 |
| 3 | 1.250 | 2.50 |
| 4 | 0.625 | 1.25 |
| 5 | 0.312 | 0.60 |
| 6 | 0.156 | 0.30 |
| 7 | 0.000 | 0.00 |
| Legend: FBS- Fetal Bovine Serum<br>PFM- Protein Free Medium<br>SCM- Serum Containing Medium<br>* The total protein content of the fetal bovine serum is considered about 50 mg/mL. | | |

Before starting the adaptation procedure the cells should be maintained with more than 80 % of viability in T-flasks in the standard medium usually employed to culture the cells.

The adaptation process is carried out step by step following the stages described below.

i. Seed 3 wells in the six-well culture plate with recombinant cell line using the standard cell culture medium (with the initial protein concentration). The cell density should be in the range of 1 to 5 x $10^5$ cells/mL. After 48 hours a half of the supernatant is replaced by fresh protein-free medium, thus rendering a final protein concentration which is 50% of the starting condition.

ii. Each 48 hours the supernatant is completely replaced by fresh culture medium with a protein concentration which is 50% of the starting condition.

iii. Cells are grown to confluence in the medium with 50% of the initial protein concentration.

iv. Cells from step iii are seeded in at least 3 wells at a density in the range 1 to 5 x $10^5$ cells/mL in culture medium with a protein concentration which is 50% of the starting condition. After 48 hours a half of the supernatant is replaced by fresh protein-free medium, rendering a final protein concentration which is 50% of the former condition.

v. Each 48 hours the supernatant is completely replaced by fresh culture medium with a protein concentration which is 50% of the former condition.

vi. Cells are grown to confluence in medium containing 50% of the previous protein concentration.

vii. Steps from (iv) to (vi) are repeated, during each cycle the protein concentration is reduced to 50% of the concentration of the previous cycle. This procedure is repeated up to reach a protein concentration which causes cell death.

viii. Cells are seeded from a cell culture with a viability of 80% or higher growing in the pre-critical protein concentration in at least 3 wells at a density in a range of 2 to 6 x $10^5$ cells/mL. Cells are grown in the pre-critical protein concentration and after 48 hours, 25% of the supernatant is replaced by fresh protein-free medium, thus rendering a final protein concentration which is the 75% of the pre-critical protein concentration.

ix. Each 48 hours the supernatant is completely replaced by fresh culture medium with a protein concentration which is 75% of the pre-critical protein concentration.

x. Cells are grown to confluence in medium containing 75% of the pre-critical protein concentration.

xi. Cell lines derived from the previous step are seeded in at least 3 wells at a density in the range 2 to 6 x $10^5$ cells/mL in medium containing 75% of the pre-critical protein concentration and after 48 hours, 25% of the culture supernatant is replaced by fresh protein-free medium, thus rendering a final protein concentration which is 75% of the previous concentration.

xii. Each 48 hours the supernatant is completely replaced by fresh culture medium with a protein concentration which is 75% of the previous concentration.

xiii. Cells are grown to confluence in medium containing 75% of the previous protein concentration.

xiv. Steps from (xi) to (xiii) are repeated, reducing the protein concentration each cycle up to 75% of that used in the previous cycle, reaching a protein concentration such that when cells are transferred to a lower concentration they grow with a similar viability and doubling time as the original, so that the subsequent decrease of the protein concentration does not affect the viability or the doubling time.

In the procedure of the present invention the initial culture medium contains a range between 5 to 10% of fetal bovine serum.

The mammalian cell line adapted to growth in protein-free medium is a myeloma, particularly NSO cells.

The present invention also could be useful when using NSO cell line transfected with a polypeptide or a recombinant protein encoding a humanized monoclonal anti-EGFR antibody hR3, particularly when the cell line is transfected with a sequence encoding a recombinant antibody or fragments thereof. The mammalian cell lines modified by procedure of the present invention growing in protein-free medium are also disclosed.

A mammalian myeloma cell line expressing a humanized or chimeric anti-EGF receptor hR3 antibody or fragments thereof growing in protein-free medium is disclosed herein as well as the antibodies secreted by these cell lines.

The cell lines obtained by the method of the present invention grow in protein-free medium stably for at least 40 generations.

**Examples:**

**Example 1:** Adaptation of recombinant cell line hR3 to protein-free medium.

The recombinant cell line hR3 was obtained by transfection of the myeloma NSO cell line with the vector constructions to express the light and heavy chains of the humanized anti-EGF human receptor hR3 monoclonal antibody.

The adaptation of this cell line to protein-free medium was carried out following the procedure Previously described, by two stage reduction of protein content of the medium.

These cells were cultured in RPMI-1640 medium supplemented with 10 % of FBS. The FBS was replaced by adding the protein reach supplement, Nutridoma NS (Boheringer Manheinn) to RPMI-1640 protein-free medium when the protein concentration was 0.15 mg/mL. The reduction of the protein content in the initial medium was done by successive dilutions with PFHM-II protein-free medium (Gibco).

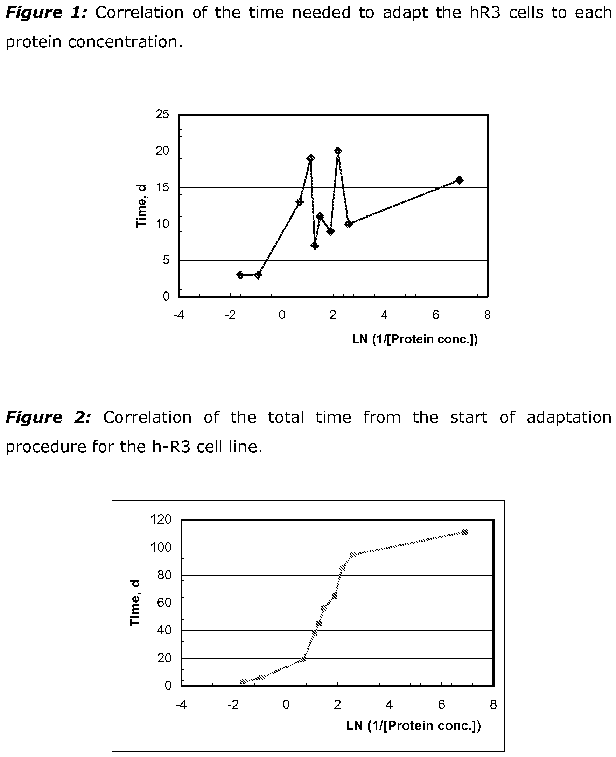

Fig. 1 and 2 respectively show the results of calculating the critical concentrations and adaptation rates $V_{adapt}$.

**Reference Example 2:** Adaptation of recombinant cell line T1hT to protein-free medium.

The recombinant cell line T1hT was obtained by transfection of the myeloma NSO cell line with the vector constructions to express the light and heavy chains of a humanized by epitope T suppression method antihuman CD6 monoclonal antibody.

The adaptation of this cell line to protein/free medium was carried out following the procedure described in the point 2, by two stage reduction of protein content of the medium.

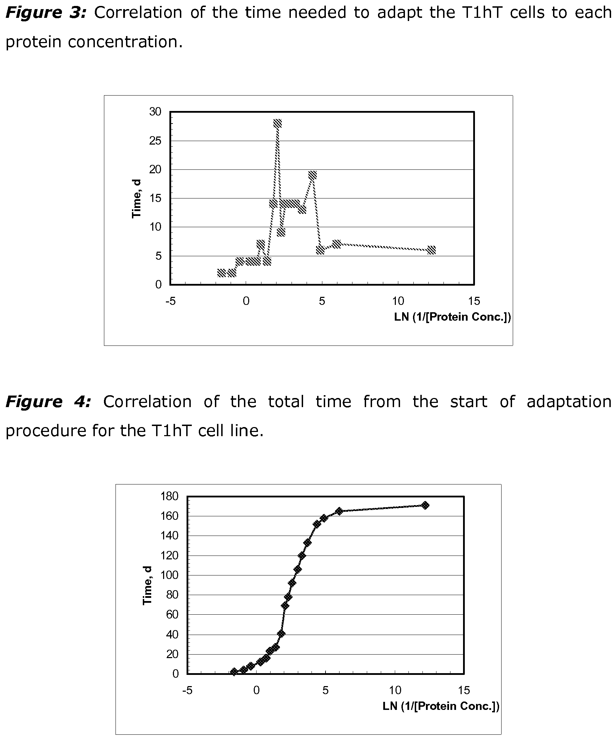

These cells were initially cultured in RPMI-1640 medium supplemented with 10 % of FBS. The reduction in the protein content was done by successive dilution of the initial medium with PFHM-II protein-free medium from Gibco. The results of calculation of the critical concentrations and adaptation rates $V_{adapt}$. are showed in the Fig. 3 and 4 respectively.

**Reference Example 3:** Adaptation of recombinant cell line T3Q to protein-free medium.

The recombinant cell line T3Q was obtained by transfection of the myeloma NSO cell line with the vector constructions to express the light and heavy chains of the humanized monoclonal antibody T3Q which recognize the CD3 receptor on human lymphocytes.

The adaptation of this cell line to protein/free medium was carried out following the procedure described in the point 2, by two stage reduction of protein content of the medium.

These cells were initially cultured in RPMI-1640 medium supplemented with 10 % of FBS. The reduction in the protein content was done by successive dilution in the PFHM-II protein-free medium from Gibco.

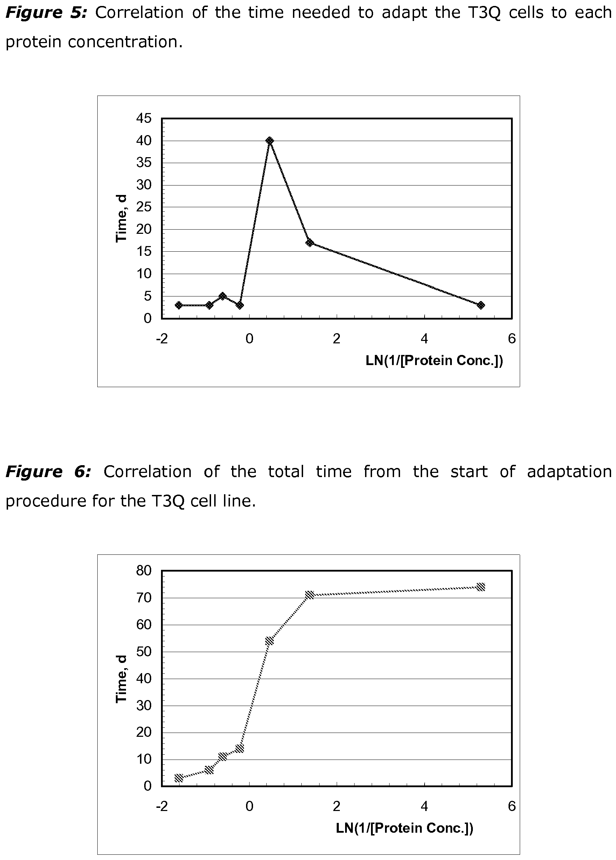

The results of calculation of the critical concentrations and adaptation rates $V_{adapt}$. are showed in the Fig. 5 and 6 respectively.

**Brief Description of the figures:**

*Figure 1:* Correlation of the time needed to adapt the hR3 cells to each protein concentration (up to recover of the viability and doubling time) with the natural logarithm of the inverse of protein concentration. Values of critical concentrations for h-R3 cell line: - 0.32 and 0.11 mg/mL of total protein concentration.

*Figure 2:* Correlation of the total time from the start of adaptation procedure with the natural logarithm of the inverse of protein concentration for the h-R3 cell line. Values of adaptation rates for h-R3 cell line during critical stage - 0.0053 mg/d.

*Figure 3:* Correlation of the time needed to adapt the T1hT cells to each protein concentration (up to recovered he viability and doubling time) with the natural logarithm of the inverse of protein concentration. Values of critical concentrations for T1hT cell line: - 0.12 and 0.01 mg/mL of total protein concentration.

*Figure 4:* Correlation of the total time from the start of adaptation procedure with the natural logarithm of the inverse of protein concentration for the T1hT cell line. Values of adaptation rates for T1hT cell line - 0.0014 mg/d.

*Figure 5:* Correlation of the time needed to adapt the T3Q cells to each protein concentration (after recovering of the viability and doubling time) with the natural logarithm of the inverse of protein concentration. Values of critical concentrations for T3Q cell line: - 0.63 mg/mL of total protein concentration.

*Figure 6:* Correlation of the total time from the start of adaptation procedure with the natural logarithm of the inverse of protein concentration for the T3Q cell line. Values of adaptation rates for T3Q cell line - 0.0172 mg/d.

Aspects and features of the present disclosure are set out in the following numbered clauses which contain the subject matter of the claims of the parent application as filed.

1.- A method for obtaining a mammalian cell line adapted to growth in a serum and protein free media, which comprises two stages:

I. A first stage wherein the cell line viability is between 80 and 100% and cells are grown in culture media with consecutive protein concentration reduction up to a critical protein concentration at which cell viability drop to 0%.
II. A second stage wherein once the critical concentration has been predetermined, then the pre-critical concentration is fixed as such protein concentration in which cellular growth is possible and it is the start point to slowly reduce the protein concentration up to the cell culture reaches the initial cellular viability and population doubling time

2.- The method according to clause 1 wherein the first stage is composed by the following steps:

i. Seed 3 wells in the six-well culture plate with recombinant cell line using the standard cell culture medium (with the initial protein concentration). The cell density should be in the range of 1 to $5 \times 10^5$ cells/mL. After 48 hours a half of the supernatant is replaced by fresh protein-free medium, thus rendering a final protein concentration which is 50% of the starting condition.
ii. Each 48 hours the supernatant is completely replaced by fresh culture medium with a protein concentration which is 50% of the starting condition.
iii. Cells are grown to confluence in the medium with 50% of the initial protein concentration.
iv. Cells from step iii are seeded in at least 3 wells at a density in the range 1 to $5 \times 10^5$ cells/mL in culture medium with a protein concentration which is 50% of the starting condition. After 48 hours a half of the supernatant is replaced by fresh protein-free medium, rendering a final protein concentration which is 50% of the former condition.
v. Each 48 hours the supernatant is completely replaced by fresh culture medium with a protein concentration which is 50% of the former condition.
vi. The cells are grown to confluence in medium containing 50% of the previous protein concentration.
vii. Steps from (iv) to (vi) are repeated, during each cycle the protein concentration is reduced to 50% of the concentration of the previous cycle. This procedure is repeated up to reach a protein concentration which causes cell death.

3.- The method according to clause 1 wherein the second stage is composed by the following steps:

viii. Cells are seeded from a cell culture with a viability of 80% or higher growing in the pre-critical protein concentration in at least 3 wells at a density in a range of 2 to $6 \times 10^5$ cells/mL. Cells are grown in the pre-critical protein concentration and after 48 hours the 25% of the supernatant is replaced by fresh protein-free medium, thus rendering a final protein concentration which is the 75% of the pre-critical protein concentration.
ix. Each 48 hours the supernatant is completely replaced by fresh culture medium with a protein concentration which is 75% of the pre-critical protein concentration.
x. Cells are grown to confluence in medium containing 75% of the previous protein concentration.
xi. Cell lines derived from the previous step are seeded in at least 3 wells at a density in the range 2 to $6 \times 10^5$ cells/mL in medium containing 75% of the pre-critical protein concentration and after 48 hours, 25% of the culture supernatant is replaced by fresh protein-free medium, thus rendering a final protein concentration which is 75% of the previous concentration.
xii. Each 48 hours the supernatant is completely replaced by fresh culture medium with a protein concentration which is 75% of the previous.
xiii. Cells are grown to confluence in medium containing 75% of the previous protein concentration.
xiv. Steps from (xi) to (xiii) are repeated, reducing the protein concentration each cycle up to 75% of that used in the previous cycle, reaching a protein concentration such that when cells are transferred to a lower concentration they grow with a similar viability and doubling time as the original, so that the subsequent decrease of the protein concentration does not affect the viability or the doubling time.

4.- The method according clauses 1 to 3 wherein the serum and protein-containing medium in which the cells are initially seeded comprises between 5% and 10% of fetal bovine serum.

5.- The method according clauses 1 to 4 wherein the mammalian cell line adapted to growth in a serum and protein free media is a myeloma.

6.- The method according clause 5 wherein the myeloma is the NSO cell line.

7.- The method according clause 6 wherein said NSO cell line contains a sequence encoding a recombinant polypeptide or a recombinant protein.

8.- The method according clause 7 wherein the sequence encoding a recombinant polypeptide or a recombinant protein codifies for a recombinant antibody or a fragment thereof.

9.- A mammalian cell line obtained by the method of clause 1 to 8, wherein said cell line is adapted to growth in a serum and protein free media.

10.- A mammalian cell line according clause 9 wherein said cell line is a myeloma.

11.- A mammalian cell line according clause 10 wherein said cell line is the NSO cell line.

12.- A mammalian cell line according clause 11 wherein the NSO cell line contains a sequence encoding a recombinant polypeptide or a recombinant protein.

13.- A mammalian cell line according clause 12 wherein the sequence encoding a recombinant polypeptide or a recombinant protein codifies for a recombinant antibody or a fragment thereof.

14.- A mammalian cell line according clause 13 wherein the sequence codifies for the humanized recombinant antibody anti-EGF-R hR3 or a fragment thereof.

15.- A mammalian cell line according clause 13 wherein the sequence codifies for the humanized recombinant anti-CD6 antibody T1hT or a fragment thereof.

16.- A mammalian cell line according clause 13 wherein the sequence codifies for the chimeric recombinant anti-CD3 antibody T3Q or a fragment thereof.

17.- Use of the method according to clauses 1 to 8 for obtaining a mammalian cell line adapted to growth in a serum and protein free media.

18.- The humanized monoclonal antibody against EGF-R hR3 or fragments thereof secreted by the cell lines obtained by the method of clauses 1 to 8.

19.- The humanized monoclonal antibody against CD6 antigen T1hT or fragments thereof secreted by the cell lines obtained by the method of clauses 1 to 8.

20.- The chimeric monoclonal antibody against CD3 antigen T3Q or fragments thereof secreted by the cell lines obtained by the method of clauses 1 to 8.

**Claims**

1. A method for producing humanized monoclonal anti-EGFR antibody hR3 or fragments thereof in serum- and protein-free media comprising adapting a recombinant mammalian myeloma cell line transfected with a sequence encoding said humanized monoclonal anti-EGFR antibody hR3 or fragments thereof, to growth in serum- and protein-free media by a method comprising:

   I. A first stage wherein the cell line viability is between 80 and 100% and cells are grown in culture media with consecutive protein concentration reduction up to a critical protein concentration at which cell viability drops to 0%; and
   II. a second stage wherein once the critical concentration has been predetermined, then the pre-critical concentration is fixed as such a protein concentration in which cellular growth is possible and is the start point to slowly reduce the protein concentration until the cell culture reaches the initial cellular viability and population doubling time.

2. The method according to claim 1, wherein the myeloma cell line is the NSO cell line.

3. The method of claim 1 or claim 2, wherein the method for producing humanized monoclonal anti-EGFR antibody hR3 or fragments thereof further comprises the step of obtaining the humanized monoclonal anti-EGFR antibody hR3 or fragments thereof.

**Patentansprüche**

1. Verfahren zur Herstellung von humanisiertem monoklonalen Anti-EGFR-Antikörper hR3 oder Fragmenten davon in Serum- und Protein-freien Medien, umfassend das Anpassen einer rekombinanten Säugetier-Myelom-Zelllinie, die mit einer Sequenzkodierung des besagten humanisierten monoklonalen Anti-EGFR-Antikörpers hR3 oder Fragmenten davon transfiziert ist, an das Wachstum in Serum- und Protein-freien Medien durch ein Verfahren welches umfasst:

   I. Eine erste Stufe, wobei die Zelllinienviabilität zwischen 80 und 100 % liegt und Zellen in Kulturmedien mit aufeinanderfolgender Proteinkonzentrationssenkung bis zu einer kritischen Proteinkonzentration wachsen, bei der die Zellviabilität auf 0 % sinkt; und
   II. eine zweite Stufe, wobei, sobald die kritische Konzentration bestimmt wurde, die vor-kritische Konzentration als solch eine Proteinkonzentration festgelegt wird, bei der Zellwachstum möglich ist und der Startpunkt ist, die Proteinkonzentration langsam zu reduzieren, bis die Zellkultur die ursprüngliche zelluläre Viabilität und Bestandsverdopplungszeit erreicht.

2. Verfahren nach Anspruch 1, wobei die Myelom-Zelllinie die NSO-Zelllinie ist.

3. Verfahren nach Anspruch 1 oder Anspruch 2, wobei das Verfahren zur Herstellung von humanisiertem monoklonalen Anti-EGFR-Antikörper hR3 oder Fragmenten davon den weiteren Schritt des Erhaltens des humanisierten monoklonalen Anti-EGFR-Antikörpers hR3 oder Fragmenten davon, umfasst.

**Revendications**

1. Procédé de production de l'anticorps monoclonal humanisé anti-récepteur de l'EGF hR3 ou des fragments de celui-ci dans un milieu exempt de sérum et de protéines, consistant à adapter une lignée cellulaire de myélome mammifère recombinante, transfectée par une séquence codant ledit anticorps monoclonal humanisé anti-récepteur de l'EGF hR3 ou des fragments de celui-ci, pour se développer dans un milieu exempt de sérum et de protéines par un procédé comprenant :

   I. une première étape dans laquelle la viabilité de la lignée cellulaire est entre 80 et 100 % et les cellules sont développées dans un milieu de culture avec une réduction consécutive de la concentration de protéines jusqu'à une concentration de protéines critique à laquelle la viabilité cellulaire tombe à 0 % ; et
   II. une seconde étape dans laquelle une fois que la concentration critique a été prédéterminée, la concentration pré-critique est alors fixée de telle sorte qu'une concentration de protéines pour laquelle la croissance cellulaire est possible et est le point de départ pour réduire lentement la concentration de protéines jusqu'à ce que la culture cellulaire atteigne la viabilité cellulaire initiale et une durée de doublement de population.

2. Le procédé selon la revendication 1, dans lequel la lignée cellulaire de myélome est la lignée cellulaire NSO.

3. Le procédé selon la revendication 1 ou la revendication 2, dans lequel le procédé de production de l'anticorps monoclonal humanisé anti-récepteur de l'EGF hR3 ou des fragments de celui-ci comprend en outre l'étape d'obtention de l'anticorps monoclonal humanisé anti-récepteur de l'EGF hR3 ou des fragments de celui-ci.

**Figure 1:** Correlation of the time needed to adapt the hR3 cells to each protein concentration.

**Figure 2:** Correlation of the total time from the start of adaptation procedure for the h-R3 cell line.

**Figure 3:** Correlation of the time needed to adapt the T1hT cells to each protein concentration.

**Figure 4:** Correlation of the total time from the start of adaptation procedure for the T1hT cell line.

*Figure 5:* Correlation of the time needed to adapt the T3Q cells to each protein concentration.

*Figure 6:* Correlation of the total time from the start of adaptation procedure for the T3Q cell line.

**REFERENCES CITED IN THE DESCRIPTION**

**Patent documents cited in the description**

- US 5891996 A
- WO 9719111 A
- WO 9705240 A
- JP 2696001 B
- WO 9626266 A
- US 5393668 A
- WO 9615231 A

**Non-patent literature cited in the description**

- **GAVILONDO et al.** *Hybridoma,* 1999, vol. 9 (5
- **ITO et al.** *PNAS U.S.A.,* 1996, vol. 93, 3598-3601
- **REITER et al.** *Cytotechnology,* 1992, vol. 9, 247-253
- **PATERSON et al.** *Appl. Microbiol. Biotechnol.,* 1994, vol. 40, 691-658